# EUROPEAN PATENT APPLICATION

(11) **EP 3 674 692 A1**
(43) Date of publication of application: **01.07.2020**
(21) Application number: 19220046.7
(22) Date of filing: 30.12.2019
(51) Int. Cl.: G01N 21/45, A61B 5/00

(54) **DEVICE FOR THE DETECTION OF BIOLOGICALLY ACTIVE MOLECULES**

(30) Priority: 31.12.2018 EP 18215943
(71) Applicant: SDS Optic Spolka Akcyjna, 20-708 Lublin (PL)
(72) Inventor: Staniszewska, Magdalena, 21-030 Motycz (PL); Staniszewski, Marcin, 21-030 Motycz (PL)
(74) Representative: Kondrat, Mariusz

(57) **Abstract**

The subject of the invention is a device comprising a fibre-optic sensor for the detection of biologically active molecules, characterised in that the fibre-optic sensor is placed in a capillary closed on one side (1) whose one-sided closure is a light-permeable material (2), the outer face (3) of which is coated with an analyte-sensitive material (8) and the inner face (4) of the light-permeable material (2) is separated from the face of the optical fibre (5) from 0 µm to 30 mm.

## Description

The subject of the invention is a device composed of a fibre-optic sensor for detection of biologically active molecules.

The state of the art describes a biosensor-based toxin detection device for milk samples in which the surface of a Fabry-Perot interferometer, which is a capillary-flow glass microplate, has been coated with fragments of antibodies, the so-called Fab' (Chalyan T et al., Asymmetric Mach-Zehnder Interferometer Based Biosensors for Aflatoxin M1 Detection, Biosensors 2016, 6, 1; doi:10.3390/bios6010001).

The US 10/483,586 application (publication no. US20040186359 A) describes a diagnostic apparatus using detection by means of a coated optical fibre; which, however, requires implantation in the body.

The Clinical Chemistry publication of 1991, entitled Evaluation of the Fiber-Optic Antibody-Based Fluoroimmunosensor for DNA Adducts in Human Placenta Samples by T. Vo-Dinh, J. P. Alarie, R. W. Johnson, M. J. Sepaniak and R. M. Santella describes the measurement of DNA adducts by means of fluorescent signal detection by a single optical fibre with antibodies placed in a pocket at the end of the fibre. The device uses laser light to excite and detect the fluorescent signal. The measurement of adducts requires preparation of the sample by hydrolysis in order to achieve the required sensitivity.

Similarly, the apparatus and method of measurement presented in Biosensors and Bioelectronics 2007, Chemiluminescent optical fiber immunosensor for detection of autoantibodies to ovarian and breast cancer-associated antigens by Orly Salama, Sebastien Herrmann, Alina Tziknovsky b, Benjamin Piura, Michael Meirovich, Ilya Trakht, Brent Reed, Leslie I. Lobel, Robert S. Marks, requires a marker, e.g. a chemiluminescent one and secondary antibodies for detection. The device is prepared to perform measurements in a solution.

The document Nature Biotechnology 2000, Antibody-based nanoprobe for measurement of a fluorescent analyte in a single cell by Tuan Vo-Dinh, Jean-Pierre Alarie, Brian M. Cullum, and Guy D. Griffin, describes another apparatus using an optical fibre coated with antibodies, suitable for use in a single cell. In this device, however, measurement is also based on detection of the fluorescent signal and the device includes a single optical fibre connected to a fluorescence microscope.

The state of the art has already indicated some solutions enabling elimination of the use of markers for detection. The Scientific Reports publication of 2014, entitled Nanoscale Label-free Bioprobes to Detect Intracellular Proteins in Single Living Cells by Wooyoung Hong, Feng Liang, Diane Schaak, Marko Loncar and Qimin Quan, describes an apparatus using an antibody-coated optical fibre that does not require fluorescent markers and is based on the surface plasmon resonance technique. The device has been adapted to perform measurements in a single cell and includes an optical fibre terminated with an antibody-coated gold nanotube. When the analyte is connected, the LSPR signal is shifted. Similarly, a Biosensors and Bioelectronics document of 2014, entitled An enhanced LSPR fiber-optic nanoprobe for ultrasensitive detection of protein biomarkers by Mollye Sanders, Yongbin Lin, Jianjun Wei, Taylor Bono, Robert G. Lindquist describes an analogous solution, also based on an optical fibre probe and the use of the LSPR signal from an antibody-coated gold nanodisc at the end of the probe. The device is adapted to determine the analyte in a solution. There are no solutions described to carry out measurements directly in the tissue.

Currently known solutions do not enable a simple, minimally invasive determination of analytes directly in tissues, including solid tissues, among others, a solid tumour, whether isolated or in situ directly in the patient, without having to use any markers and without prior tissue preparation. Current diagnostic solutions require sampling, fixation and/or processing and therefore do not reflect the actual condition of the tissue. In addition, devices using the flow of the tested substance through the device (as in the case of microplates) require significant amounts of the sample. The use of a microprobe used for in-situ measurements ensures safety in use and limits the amount of material required for the assay.

A device known from the description of the P.420189 application contains an optical sensor that uses selective interaction with the tested substance of the binding agents immobilised on the surface of the optical fibre which is the interferometer arm, where the optical fibre being the interferometer arm, containing the immobilized binding agent, is fixed inside a guide, preferably a metal one, enabling piercing of the tissue and direct measurement of the substance present in the tissue, so as to detect it and/or determine the concentration. Design of the device, according to the invention, enables to insert a fibre-based sensor without interfering with the measurement and damaging the sensor itself, directly into the tissue. This device also enables in-situ measurement with an optical fibre-based probe in a safe and minimally invasive way for the patient. The guide also makes it possible to protect the patient from the remaining part of the fibre-optic sensor in the tissue, e.g. should the sensor accidentally crack or break. The optical fibre may be fixed in the guide and thus shielded in part or over its entire length to reduce the effect of any shocks on the measurement result.

The purpose of the invention was to provide a new device containing a fibre-optic sensor.

The essence of the invention is a device for detecting biologically active molecules, consisting of a fibre-optic sensor, characterized by the fact that the fibre-optic sensor is placed in a capillary closed on one side, the closure being a light-permeable material, whose external front surface is covered with an analyte-sensitive material, while the internal front surface of the light-permeable material is distant from the front of the fibre-optic cable between 0 µm to 30 mm.

Preferably, the capillary has an internal diameter between 1 micrometer and 3.00 mm and an external diameter between 2 micrometers and 5.00 mm

Preferably, the light-permeable material is a material selected from the group including a disc, a quartz plate, a glass plate or a plastic plate.

Preferably the front face of the optical-fibre touches the internal face of the light-permeable material.

Preferably, the light-permeable material is between 1 µm and 3 mm thick.

Preferably, the fibre-optic sensor is mounted in the capillary so that the outer wall of sensor 6 is located away from the inner wall of capillary 7 in the range from 0 µm to 1.499 mm.

Preferably the fibre-optic sensor is made rigid relative to the capillary near the open end of the capillary by means of glue for medical applications or a weld.

The invention provides the following advantages:
The preferable effect is obtained through the use of a light-permeable material (e.g. a transparent disc) closing one end of the capillary as a resonance cavity to produce an interference phenomenon for the reflective interferometer

The invention is shown in figures, where fig. 1 shows the top view of the capillary; fig. 2 shows the bottom view of the capillary; fig. 3 is a schematic representation of the fibre-optic sensor according to the invention in an embodiment in which the fibre-optic face contacts the inner face of the light-permeable material; fig. 4 is a schematic representation of the fibre-optic sensor according to the invention in an embodiment in which the fibre-optic face is distant from the inner face of the light-permeable material; fig. 5 is a schematic representation of the fibre-optic sensor according to the invention, taking into account the example parameters of the capillary inner wall; fig. 6 is a schematic representation of the fibre-optic sensor according to the invention including the analyte-sensitive material; fig. 7 shows the optical fibre attached to the other end of the capillary.

The invention is presented in embodiments.

### Embodiment 1

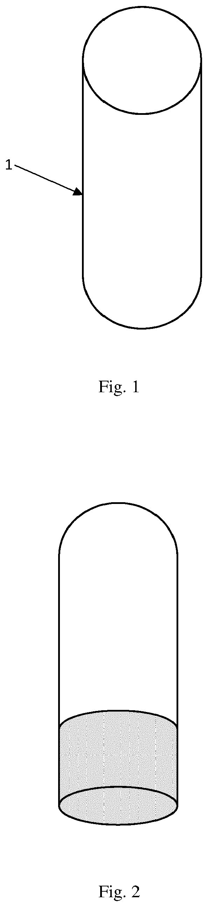

The apparatus according to the invention containing the fibre-optic sensor is shown in fig. 1 to 7, where 1 is the capillary; 2 is the light-permeable material; 3 is the outer face of the light-permeable material; 4 is the inner face of the light-permeable material; 5 is the face of the optical fibre; 6 is the outer face of the sensor; 7 is the inner face of the capillary and 8 is the analyte-sensitive material.

Where in the apparatus, according to the invention, the fibre-optic sensor is placed in a capillary 1 closed on one side, where the one-sided closure is the light-permeable material 2, whose outer face 3 is covered with an analyte-sensitive material and the inner face 4 of the light-permeable material 2 is distant from the front of the fibre-optic 5 between 0 µm to 30 mm.

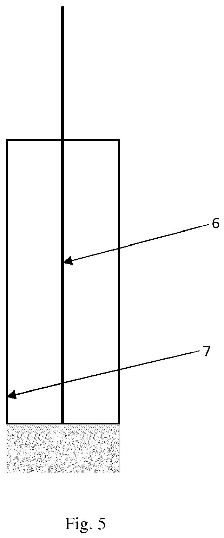

The capillary (fig. 1-2) is a tube open at one end and closed at the other end. In this embodiment, the capillary is made of plastic or metal or glass with external dimensions: internal diameter between 1 µm and 3.00 mm and external diameter between 2 µm and 5.00 mm (fig. 5).

Capillary 1 can be made by means of a device such as the Microelectrode Puller from World Precision Instruments. The fabrication of the capillary uses processes such as drawing, heating, cooling.

One end of the capillary 1 is closed by a light-permeable material 2. Whereas the light-permeable material 2 is a material selected from the group including a disc, a quartz plate, a glass plate or a plastic plate. Whereas the light-permeable material 2 is between 1 µm and 3 mm thick.

In this embodiment, one end of capillary 1 is closed by a disc made of plastic or glass, through which light can pass. The disc can be for example welded to capillary 1 with the GPX-3400 fusion splicer. The diameter of the disc is equal to the outer diameter of capillary 1 or greater in the range from 0.0 mm to 5 mm. The disc acts as for example the sensor's resonance cavity (fig. 2).

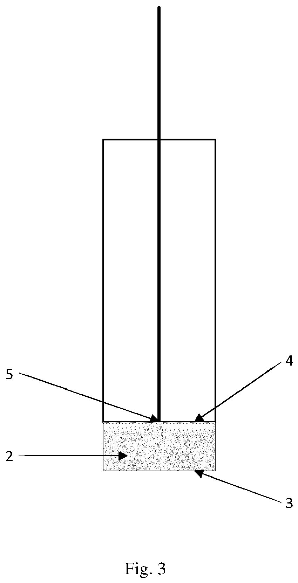

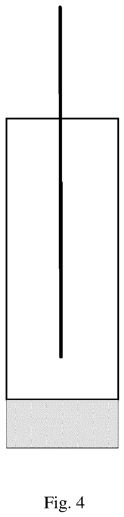

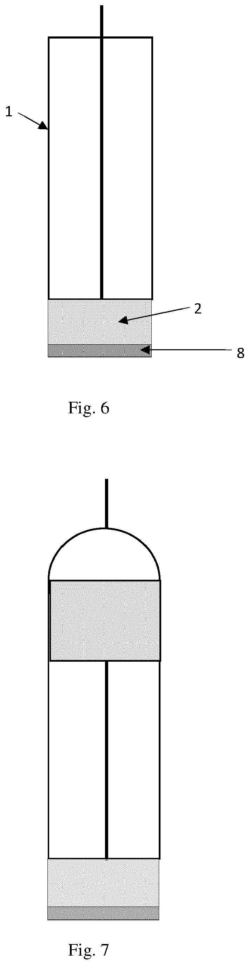

In capillary 1 there is an optical fibre that face 5 at one end is in contact with the inner face 4 of the welded disc (fig. 3) or it is located at a distance of 0.0 mm to 30.0 mm from the inner face of the disc 4 (fig. 4). The other end of the fibre is attached to the other end of the capillary and is further free. Preferably, the other end of the optical fibre of the sensor according to the invention is made rigid against the capillary (1) at the open end of the capillary (1) with an adhesive for medical applications or a weld (fig. 7).

In this embodiment the outer wall of sensor 6 is located away from the inner wall of capillary 7 in the range from 0 µm to 1.499 mm.

In this embodiment the thickness of the light-permeable disc is between 1 µm and 3.00 mm. The disc can be made e.g. in such a way that a tube made of transparent material e.g. glass, plastic, quartz is welded to the capillary e.g. with a GPX-3400 fusion splicer and then cut to length with a fibre-optic cutter e.g. LDC-400.

The disc is coated on the outside with an analyte-sensitive material 8 e.g. with substances sensitive to the biological or chemical agent being tested, in the same way as the fibre-optic sensor head in application PCT_EN2017_050030 (fig. 6).

The disc at the end of the capillary forms a resonance cavity which results in the structure of a reflective interferometer, where the reference beam is the beam reflected from the front of the optical fibre and the measuring beam is the beam reflected from the test substance (analyte) that has deposited on the front of the sensitive disc (covered with a suitable analyte-sensitive material such as the front of a fibre-optic sensor in application PCT_EN2017_050030).

The interference occurs between two beams: the beam reflected from the front face of the fibre at the fibre/disc boundary and the beam reflected from the disc/analyte at the analyte settling point. The use of e.g. polarized fibre limits the influence of external factors, especially temperature changes on the polarization of the beam, i.e. possible fluctuations in the interference contrast.

## Claims

1. A device for the detection of biologically active molecules comprising a fibre-optic sensor, **characterised in that** the fibre-optic sensor is placed in a capillary closed on one side (1) that one-sided closure is a light-permeable material (2), the outer face (3) of which is coated with an analyte-sensitive material (8) and the inner face (4) of the light-permeable material (2) is separated from the face of the optical fibre (5) from 0 µm to 30 mm.

2. The device according to claim 1 **characterized in that** the capillary (1) has an internal diameter between 1 µm and 3.00 mm and an external diameter between 2 µm and 5.00 mm.

3. The device according to claim 1 or 2 **characterized in that** the light-permeable material (2) is a material selected from the group including a disc, a quartz plate, a glass plate or a plastic plate.

4. The device according to any of the previous claims from 1 to 3, **characterized in that** the face of the optical fibre (5) touches the inner face (4) of the light-permeable material (2).

5. The device, according to any of the previous claims 1 to 4, **characterized in that** the light-permeable material (2) is from 1 µm to 3 mm thick.

6. The device according to any of the previous claims 1 to 5, **characterized in that** the fibre-optic sensor is installed in the capillary (1), so that the external wall of sensor 6 is located away from the internal wall of the capillary 7 in the range from 0 µm to 1.499 mm.

7. The device according to any of the previous claims from 1 to 6, **characterized in that** the fibre-optic sensor is made rigid relative to the capillary (1) at the open end of the capillary (1) with glue for medical applications or a weld.
